# EUROPEAN PATENT APPLICATION

(11) **EP 3 488 843 A1**
(43) Date of publication of application: **29.05.2019**
(21) Application number: 17831403.5
(22) Date of filing: 24.07.2017
(51) Int. Cl.: A61K 8/02, A61K 8/31, A61K 8/92, A61K 8/89, A61K 8/29, A61K 8/25, A61K 8/19, A61K 8/34, A61K 8/06, A61Q 19/00

(54) **COSMETIC PRODUCT INCLUDING COSMETIC CONTAINER USING SPECIFIC RUBBER**

(30) Priority: 22.07.2016 KR 20160093214
(71) Applicant: Cosmax, Inc., Hwaseong-si, Gyeonggi-do 18622 (KR)
(72) Inventor: JO, Na Rae, Yongin-si Gyeonggi-do 16838 (KR); KIM, Sung Yong, Anyang-si Gyeonggi-do 14000 (KR); YOO, Kweon Jong, Suwon-si Gyeonggi-do 16332 (KR); PARK, Myeong Sam, Seoul 04987 (KR); PARK, Chun Ho, Yongin-si Gyeonggi-do 17081 (KR); PARK, Sung Mi, Gyeonggi-do 18476 (KR)
(74) Representative: Mammel und Maser
(86) International application number: PCT/KR2017/007959
(87) International publication number: WO 2018/016931

(57) **Abstract**

Disclosed is a cosmetic product including: a cosmetic composition; and a cosmetic container in which the cosmetic composition is filled, wherein the cosmetic container includes nitrile butadiene rubber and has pores of which the average size is about 0.2 mm to about 1 mm, and the cosmetic composition has a viscosity of about 2,000 cps to about 10,000 cps.

## Description

### [Technical Field]

The present application relates to a cosmetic product including: a cosmetic composition; and a cosmetic container in which the cosmetic composition is filled, wherein the cosmetic container includes nitrile butadiene rubber and has pores of which the average size is about 0.2 mm to about 1 mm, and the cosmetic composition has a viscosity of about 2,000 cps to about 10,000 cps.

### [Background Art]

In general, a cushion pact is a base makeup cosmetic product which decorates the face with a natural beige color, and a cosmetic composition which is a content of the base makeup cosmetic product is usually produced as a solution (liquid). The cushion pact is usually in the form of a double vessel containing an impregnation material impregnated with a cosmetic composition, a refill inner vessel which has the impregnation material impregnated with the cosmetic composition therein, and an external vessel capable of engaging and receiving the refill inner container.

As an impregnation material used in a cosmetic product in the related art, a polyurethane foam is usually used, and the polyurethane foam is divided into a polyether polyol foam and a polyester polyol foam according to the type of polyol used for a polymer polymerization reaction. Korean Patent No. 10-1257628 discloses a cosmetic product including a foamed urethane foam having an ether polymer-type network structure impregnated with a cosmetic composition.

However, for the polyurethane foam, the amount of content discharged and stability may not be uniform. Further, constituent components of the polyurethane foam react with active materials such as silicone oil and a UV blocking composition, and as a result, there is a problem with durability, such as swelling or melting of the polyurethane foam as time elapses.

### [Disclosure]

### [Technical Problem]

The present application has been made in an effort to provide a cosmetic product including: a cosmetic composition; and a cosmetic container in which the cosmetic composition is filled, wherein the cosmetic container includes nitrile butadiene rubber and has pores of which the average size is about 0.2 mm to about 1 mm, and the cosmetic composition has a viscosity of about 2,000 cps to about 10,000 cps.

However, a technical problem to be achieved by the present invention is not limited to the aforementioned problem, and the other problems that are not mentioned may be clearly understood by the person skilled in the art from the following description.

### [Technical Solution]

A first aspect of the present application provides a cosmetic product including: a cosmetic composition; and a cosmetic container in which the cosmetic composition is filled, wherein the cosmetic container includes nitrile butadiene rubber and has pores of which the average size is about 0.2 mm to about 1 mm, and the cosmetic composition has a viscosity of about 2,000 cps to about 10,000 cps.

### [Advantageous Effects]

In exemplary embodiments of the present application, by using a cosmetic container including a specific nitrile butadiene rubber, the feeling of use is softer than when an existing cushion pact is used, the cosmetic composition may be uniformly discharged in a constant amount, and it is also possible to provide a cosmetic container having excellent chemical resistance, hydrolysis resistance and/or durability.

In exemplary embodiments of the present application, in the case of using a cosmetic container according to an exemplary embodiment of the present application, which has excellent durability, it is possible to alleviate a problem in that a foam is worn out and strength is reduced due to pressure which a user repeatedly and continuously applies to the form by using an applicator such as a hand or a puff.

In exemplary embodiments of the present application, in the case of impregnating an existing polyurethane foam with a low-viscosity cosmetic composition, there is a problem in that the storability deteriorates and the cosmetic composition flows down, but in exemplary embodiments of the present application, in the case of impregnating a cosmetic container including the specific nitrile butadiene rubber with a low-viscosity cosmetic composition, the cosmetic composition is stably supported, so that storability is excellent.

In exemplary embodiments of the present application, a cosmetic container of the present application has an excellent ability to appropriately support the cosmetic composition in the cosmetic container because the filling efficiency of the cosmetic composition is excellent.

In exemplary embodiments of the present application, when consumers use a cosmetic product of the present application, it is possible to prevent the cosmetic composition from being excessively or insufficiently discharged because a pick-up amount of cosmetic composition obtained by applying vertical pressure to the cosmetic container is constant, so that the cosmetic product is conveniently used and the cosmetic effect is excellent.

In exemplary embodiments of the present application, in the case of using the cosmetic composition having a viscosity of about 2,000 cps to about 10,000 cps for the cosmetic product of the present application, a low-viscosity cosmetic composition may be stably supported, so that the cosmetic composition does not leak out to the outside even though used for a long period of time.

In exemplary embodiments of the present application, in the case of including a hydrophilic functional group in the nitrile butadiene rubber, it is possible to prevent the cosmetic container from being excessively impregnated with the cosmetic composition, and the abrasion resistance is improved.

### [Description of Drawings]

FIGS. 1A and 1B are each photographs immediately after an NBR sponge and a polyurethane sponge in the related art are respectively impregnated with a cosmetic composition having a viscosity of about 14,500 cps in an exemplary embodiment of the present application.
FIGS. 2A and 2B are each SEM images of the cosmetic container prepared in an exemplary embodiment of the present application.
FIG. 3 is a graph illustrating a sensory comparison result of an NBR-substrate cosmetic container prepared by an exemplary embodiment of the present application and a polyurethane foam in the related art.
FIG. 4A is a photograph immediately after a polyurethane foam in the related art is impregnated with a cosmetic composition having a viscosity of about 2,000 cps to about 3,000 cps.
FIG. 4B is a photograph immediately after an NBR-substrate cosmetic container prepared by an exemplary embodiment of the present application is impregnated with a cosmetic composition having a viscosity of about 2,000 cps to about 3,000 cps.
FIG. 4C is a photograph about 12 hours after a polyurethane foam in the related art is impregnated with a cosmetic composition having a viscosity of about 2,000 cps to about 3,000 cps.
FIG. 4D is a photograph 12 hours after an NBR-substrate cosmetic container prepared by an exemplary embodiment of the present application is impregnated with a cosmetic composition having a viscosity of about 2,000 cps to about 3,000 cps.
FIG. 5A is a photograph immediately after a polyurethane foam in the related art is impregnated with a cosmetic composition having a viscosity of about 6,500 cps.
FIG. 5B is a photograph immediately after an NBR-substrate cosmetic container prepared by an exemplary embodiment of the present application is impregnated with a cosmetic composition having a viscosity of about 6,500 cps.
FIG. 5C is a photograph after a polyurethane foam in the related art is impregnated with a cosmetic composition having a viscosity of about 6,500 cps, and then is stored at about 45°C for 1 month.
FIG. 5D is a photograph after an NBR-substrate cosmetic container prepared by an exemplary embodiment of the present application is impregnated with a cosmetic composition having a viscosity of about 6,500 cps, and then is stored at about 45°C for 1 month.

### [Best Mode]

Hereinafter, examples of the present application will be described in detail such that a person skilled in the art to which the present application pertains can easily carry out the present application with reference to the accompanying drawings. However, the present application can be implemented in various different forms, and is not limited to the exemplary embodiments described herein. In addition, in order to clearly explain the present application, portions that are not related to the explanation are omitted in the drawings, and like reference numerals are added to like portions throughout the specification.

Throughout the specification of the present application, when one part is "connected" to another part, this includes not only a case where they are "directly connected to each other", but also a case where they are "electrically connected to each other" with another element therebetween.

Throughout the specification of the present application, when one member is disposed "on" another member, this includes not only a case where the one member is brought into contact with another member, but also a case where still another member is present between the two members.

Throughout the present specification, when one part "includes" one constituent element, unless otherwise specifically described, this does not mean that another constituent element is excluded, but means that another constituent element may be further included. Throughout the specification of the present application, a term of a degree, such as "about" or "substantially", is used in a corresponding numerical value or used as a meaning close to the numerical value when an unique manufacturing and material allowable error is suggested to a mentioned meaning, and is used to prevent an unconscionable infringer from illegally using disclosed contents including a numerical value illustrated as being accurate or absolute in order to help the understanding of the present application. Throughout the specification of the present application, a term of a degree, such as a "step ... " or a "step of ...", does not mean a "step for ...".

Throughout the specification of the present application, the term "combination thereof' included in the Markush type expression means a mixture or combination of one or more selected from the group consisting of constituent elements described in the Markush type expression, and means including one or more selected from the group consisting of the constituent elements.

Throughout the specification of the present application, the description "A and/or B" means "A or B, or A and B".

Throughout the specification of the present application, "an alkyl group" and "an alkylene group" each independently indicate a linear or branched alkyl group and alkylene group having 1 to 24 carbon atoms, 1 to 20 carbon atoms, 1 to 18 carbon atoms, 1 to 15 carbon atoms, 1 to 12 carbon atoms, 1 to 10 carbon atoms, 1 to 8 carbon atoms, 1 to 6 carbon atoms, 1 to 5 carbon atoms, or 1 to 3 carbon atoms. When the alkyl group or alkylene group is substituted with an alkyl group, this is used interchangeably with "a branched alkyl group" or "a branched alkylene group". A substituent which may be substituted with the alkyl group or alkylene group may include at least one selected from the group consisting of halo (for example, F, Cl, Br, and I), haloalkyl (for example, CCl₃ or CF₃), alkoxy, alkylthio, hydroxy, carboxy (-C(O)-OH), alkyloxy carbonyl (-C(O)-O-R), alkyl carbonyloxy (-O-C(O)-R), amino (-NH₂), carbamoyl (-C(O)NHR), urea (-NH-C(O)-NHR-), and thiol (-SH), but may not be limited thereto. Furthermore, an alkyl group having two or more carbon atoms among the above-described alkyl groups may include at least one carbon-carbon double bond or at least one carbon-carbon triple bond, but may not be limited thereto. For example, the alkyl group may include methyl, ethyl, propyl, butyl, pentyl, hexyl, hepxyl, octyl, nonyl, decyl, undecyl, dodecyl, tridecyl, tetradecyl, pentadecyl, hexadecyl, heptadecyl, octadecyl, nonadecyl, acosanyl, or all available isomers thereof, but may not be limited thereto. For example, the alkyl group used in the present application may be an alkyl group having 1 to 10 carbon atoms, that is, a methyl group, an ethyl group, a propyl group, a butyl group, a pentyl group, a hexyl group, a heptyl group, an octyl group, a nonyl group, or a decyl group, or an alkyl group having 1 to 6 carbon atoms, that is, a methyl group, an ethyl group, a propyl group, a butyl group, a pentyl group, or a hexyl group, or an alkyl group having 1 to 4 carbon atoms, that is, a methyl group, an ethyl group, an i-propyl group, an n-propyl group, a t-butyl group, an s-butyl group, or an n-butyl group, but is not limited thereto.

Throughout the specification of the present application, the "filling efficiency" means an ability of a cosmetic container to fill a cosmetic composition, and may be derived by calculating an actual filling amount of cosmetic container as compared to a target filling amount of cosmetic container.

Throughout the specification of the present application, the "pick-up amount" may mean an amount of cosmetic composition discharged by applying vertical pressure to the cosmetic container in which the cosmetic composition is filled using an applicator such as a hand or a puff.

Throughout the specification of the present application, the "size of pores" or the "average size of pores" may mean an average value of pore sizes measured by using a scanning electron microscope (SEM, manufacturer: SEC, model name: SNE-4500 M). Hereinafter, the exemplary embodiment and example of the present application will be described in detail with reference to the accompanying drawings. However, the present application may not be limited to the exemplary embodiment and example and the drawings.

A first aspect of the present application provides a cosmetic product including: a cosmetic composition; and a cosmetic container in which the cosmetic composition is filled, wherein the cosmetic container includes nitrile butadiene rubber (hereinafter, also referred to as 'NBR') and has pores of which the average size is about 0.2 mm to about 1 mm, and the cosmetic composition has a viscosity of about 2,000 cps to about 10,000 cps.

In an exemplary embodiment of the present application, the NBR may be included in an amount of about 80 parts by weight or more, about 85 parts by weight or more, about 90 parts by weight or more, about 95 parts by weight or more, about 97 parts by weight or more, about 99 parts by weight or more, or about 100 parts by weight, based on 100 parts by weight of the total weight before the cosmetic container is filled with the cosmetic composition.

In an exemplary embodiment of the present application, pores which the cosmetic container includes may have an average size of about 0.2 mm to about 1 mm. For example, pores which the cosmetic container includes may have an average size of about 0.2 mm to about 1 mm, about 0.2 mm to about 0.9 mm, about 0.2 mm to about 0.8 mm, about 0.2 mm to about 0.7 mm, about 0.2 mm to about 0.6 mm, about 0.2 mm to about 0.5 mm, about 0.2 mm to about 0.4 mm, about 0.2 mm to about 0.3 mm, about 0.3 mm to about 1 mm, about 0.3 mm to about 0.9 mm, about 0.3 mm to about 0.8 mm, about 0.3 mm to about 0.7 mm, about 0.3 mm to about 0.6 mm, about 0.3 mm to about 0.5 mm, about 0.3 mm to about 0.4 mm, about 0.4 mm to about 1 mm, about 0.4 mm to about 0.9 mm, about 0.4 mm to about 0.8 mm, about 0.4 mm to about 0.7 mm, about 0.4 mm to about 0.6 mm, or about 0.4 mm to about 0.5 mm.

In an exemplary embodiment of the present application, when pores which the cosmetic container includes have an average size of less than 0.2 mm, the space capable of accommodating the cosmetic composition is small, so that the filling efficiency may be decreased. In addition, when pores which the cosmetic container includes have an average size of more than 1 mm, the space of the pores is too large to stably store the cosmetic composition, and the ability to adjust the discharge of the cosmetic composition deteriorates, so that the pick-up amount per time may not be constant. However, the average pore size is not limited thereto.

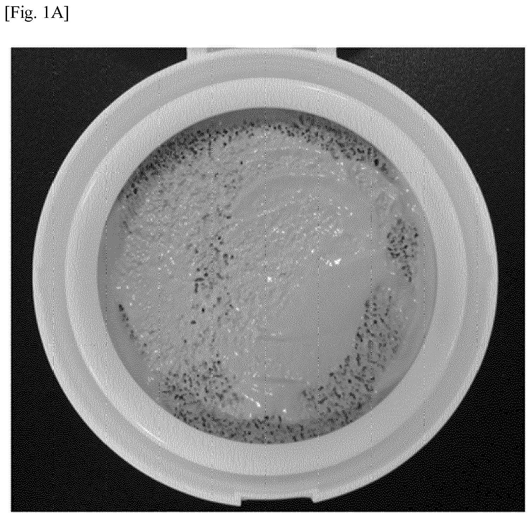

In an exemplary embodiment of the present application, the cosmetic composition may have a viscosity of about 2,000 cps to about 10,000 cps. For example, the viscosity may be about 2,000 cps to about 10,000 cps, about 2,000 cps to about 9,000 cps, about 2,000 cps to about 8,000 cps, about 2,000 cps to about 7,000 cps, about 2,000 cps to about 6,000 cps, about 2,000 cps to about 5,000 cps, about 2,000 cps to about 4,000 cps, about 2,000 cps to about 3,000 cps, about 3,000 cps to about 10,000 cps, about 3,000 cps to about 9,000 cps, about 3,000 cps to about 8,000 cps, about 3,000 cps to about 7,000 cps, about 3,000 cps to about 6,000 cps, about 3,000 cps to about 5,000 cps, about 3,000 cps to about 4,000 cps, about 4,000 cps to about 10,000 cps, about 4,000 cps to about 9,000 cps, about 4,000 cps to about 8,000 cps, about 4,000 cps to about 7,000 cps, about 4,000 cps to about 6,000 cps, about 4,000 cps to about 5,000 cps, about 5,000 cps to about 10,000 cps, about 5,000 cps to about 9,000 cps, about 5,000 cps to about 8,000 cps, about 5,000 cps to about 7,000 cps, about 5,000 cps to about 6,000 cps, about 6,000 cps to about 10,000 cps, about 6,000 cps to about 9,000 cps, about 6,000 cps to about 8,000 cps, about 6,000 cps to about 7,000 cps, about 8,000 cps to about 10,000 cps, about 8,000 cps to about 9,000 cps, or about 9,000 cps to about 10,000 cps, preferably about 3,000 cps to about 8,000 cps, and more preferably about 5,000 cps to about 8,000 cps. When the cosmetic container is impregnated with a cosmetic composition having a viscosity of less than about 2,000 cps, the cosmetic composition is excessively thin, so that the cosmetic composition may flow out without being stably supported in the cosmetic container. Furthermore, when the cosmetic container is impregnated with a cosmetic composition having a viscosity of more than about 10,000 cps, the cosmetic composition may remain outside without being impregnated into the cosmetic container, as illustrated in FIG. 1A.

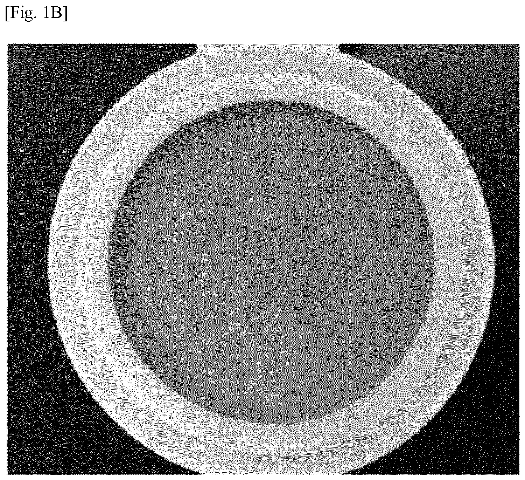

In this regard, there are problems in that when an NBR sponge in the related art is filled with a high-viscosity cosmetic composition, immediately after the filling, the sponge is not sufficiently impregnated with and the upper portion of the sponge is flooded with the cosmetic composition, or the cosmetic composition flows out, or is leaked out or discharged, as illustrated in FIG. 1A, and in order to solve these problems, the exemplary embodiments of the present application relating to a suitable range of the cosmetic composition in the cosmetic container including the nitrile butadiene rubber of the present application are provided. Specifically, it can be confirmed that when a polyurethane sponge in the related art was impregnated with a cosmetic composition having a high viscosity of about 14,500 cps, the sponge was stably impregnated (FIG. 1B), but when an NBR sponge in the related art was impregnated with a cosmetic composition having a high viscosity of 14,500 cps, the sponge was not impregnated with the cosmetic composition, and the cosmetic composition remained in the upper portion of the sponge (FIG. 1A). Accordingly, exemplary embodiments of the present application are technically characterized in that when a cosmetic container is filled with a cosmetic composition having a viscosity of about 2,000 cps to about 10,000 cps which is an optical cosmetic composition viscosity range, preferably about 3,000 cps to about 8,000 cps, or more preferably about 5,000 cps to about 8,000 cps in an NBR sponge, the NBR sponge was stably impregnated immediately after the cosmetic container was filled and during the storage for a long period of time, and as a result, the cosmetic composition does not leak out to the outside of the cosmetic container including the nitrile butadiene rubber of the present application.

In an exemplary embodiment of the present application, the nitrile butadiene rubber may include a hydrophilic functional group, but is not limited thereto. The hydrophilic functional group may be selected from the group consisting of, for example, -COOH, -SO₃, -PO₄H, -(CH₂)ₙSH (in the formula, n is an integer of 1 to 30), - ((CH₂)₂O)ₙX (in the formula, X = hydrogen or an alkyl group, and n is an integer of 2 to 5), -OH, -SH, -O(CH₂S)ₙSH (in the formula, n is an integer of 1 to 30), -NH₂, and combinations thereof, but is not limited thereto. Specifically, those including -COOH as the hydrophilic functional group may include a carboxylated nitrile butadiene rubber. In an exemplary embodiment of the present application, the hydrophilic functional group included in the nitrile butadiene rubber may be included in an amount of about 20 parts by weight or less, about 15 parts by weight or less, or about 10 parts by weight or less, based on the total weight of the cosmetic container before the cosmetic container is filled with the cosmetic composition, but the content is not limited thereto.

In an exemplary embodiment of the present application, when the carboxylated nitrile butadiene rubber is used as the nitrile butadiene rubber, a -COOH group may be included in an amount of about 20 parts by weight or less, about 15 parts by weight or less, or about 10 parts by weight or less, based on the total weight 100 parts by weight of the cosmetic container before the cosmetic container is filled with the cosmetic composition, but the content is not limited thereto.

In exemplary embodiments of the present application, in the case of including a hydrophilic functional group in the nitrile butadiene rubber, it is possible to prevent the cosmetic composition from being excessively absorbed in the cosmetic container, and the abrasion resistance may be improved.

In an exemplary embodiment of the present application, the cosmetic container may include about 10 ppi (pore per inch, the number of pores per 1 inch) to about 150 ppi of pores. For example, the cosmetic container may include about 10 ppi to about 150 ppi, about 10 ppi to about 130 ppi, about 10 ppi to about 110 ppi, about 10 ppi to about 90 ppi, about 10 ppi to about 70 ppi, about 10 ppi to about 50 ppi, about 10 ppi to about 30 ppi, about 30 ppi to about 150 ppi, about 30 ppi to about 130 ppi, about 30 ppi to about 110 ppi, about 30 ppi to about 90 ppi, about 30 ppi to about 70 ppi, about 30 ppi to about 50 ppi, about 50 ppi to about 150 ppi, about 50 ppi to about 130 ppi, about 50 ppi to about 110 ppi, about 50 ppi to about 90 ppi, about 50 ppi to about 70 ppi, about 70 ppi to about 150 ppi, about 70 ppi to about 130 ppi, about 70 ppi to about 110 ppi, about 70 ppi to about 90 ppi, about 90 ppi to about 150 ppi, about 90 ppi to about 130 ppi, about 90 ppi to about 110 ppi, about 110 ppi to about 150 ppi, about 110 ppi to about 130 ppi, or about 130 ppi to about 150 ppi of pores, but the concentration is not limited thereto.

In an exemplary embodiment of the present application, a hardness of the cosmetic container may be about 10 to about 95 based on an ASKER hardness tester type F, but is not limited thereto. For example, the hardness may be about 10 to about 95, about 10 to about 85, about 10 to about 75, about 10 to about 65, about 10 to about 55, about 10 to about 45, about 10 to about 35, about 15 to about 95, about 15 to about 85, about 15 to about 75, about 15 to about 65, about 15 to about 55, about 15 to about 45, or about 15 to 35, but is not limited thereto.

In an exemplary embodiment of the present application, the cosmetic container may include an open cell structure, but the structure is not limited thereto.

In an exemplary embodiment of the present application, the filling efficiency of the cosmetic container filled with the cosmetic composition may be about 75% or more, but is not limited thereto. For example, in the cosmetic product, the cosmetic container may include a filling efficiency of about 75% or more, about 80% or more, about 85% or more, about 90% or more, about 95% or more, about 97% or more, about 99% or more, or about 100%, but the filling efficiency is not limited thereto. The "filling efficiency" means an amount of cosmetic container filled as compared to a target filling amount of cosmetic container.

In an exemplary embodiment of the present application, the pick-up amount of cosmetic composition filling the cosmetic container may be about 0.3 g to about 0.7 g per time, but is not limited thereto. For example, the pick-up amount may be about 0.3 g to about 0.7 g, about 0.3 g to about 0.6 g, about 0.3 g to 0.5 g, about 0.4 g to about 0.7 g, about 0.4 g to about 0.6 g, or about 0.4 g to about 0.5 g, but is not limited thereto. The "pick-up amount" means an amount of cosmetic composition discharged by applying vertical pressure to the cosmetic container in which the cosmetic composition is filled using an applicator such as a hand or a puff.

In an exemplary embodiment of the present application, the applicator may include those used as a use for applying the cosmetic composition without limitation, and may include, for example, a brush, a puff, a sponge, a roller, a stick, or the like, but is not limited thereto.

In an exemplary embodiment of the present application, the cosmetic container may include those selected from the group consisting of a vulcanizing agent, a vulcanization accelerator, a foam stabilizer, a coagulant, a dispersant, and combinations thereof, but is not limited thereto.

In an exemplary embodiment of the present application, as the vulcanizing agent, a typical vulcanizing agent containing sulfur (S), selenium (Se), or tellurium (Te) may be used, but the vulcanizing agent is not limited thereto.

In an exemplary embodiment of the present application, the vulcanizing agent may be added in an amount of about 0.5 part by weight to about 1.5 parts by weight based on the total weight 100 parts by weight of the cosmetic container in the composition, but the content of the vulcanizing agent is not limited thereto. When the content of the vulcanizing agent is less than 0.5 part by weight, elastic force of the cosmetic container is lowered, and when the content is more than 1.5 parts by weight, sufficient elastic restoring force may not be possessed when constant external pressure is applied to the cosmetic container, but the content of the vulcanizing agent is not limited thereto.

In an exemplary embodiment of the present application, the vulcanization accelerator may include all types of vulcanization accelerators typically used, but is not limited thereto. For example, as the vulcanization accelerator, zinc diethyldithiocarbamate, a zinc salt of 2-mercaptobenzothiazole, EZ, MZ, or ZnO may be used, but the vulcanization accelerator is not limited thereto.

In an exemplary embodiment of the present application, the vulcanization accelerator may be added in an amount of about 0.5 part by weight to about 2 parts by weight based on the total weight 100 parts by weight of the cosmetic container in the composition, but the content of the vulcanization accelerator is not limited thereto. When the content of the vulcanization accelerator is less than about 0.5 part by weight, the vulcanization temperature and time may not be reduced, and when the content is more than about 2 parts by weight, the vulcanization process progresses excessively fast, so that a uniform cosmetic container may not be prepared, but the content is not limited thereto.

In an exemplary embodiment of the present application, the foam stabilizer may include all types of foam stabilizers typically used, but is not limited thereto. For example, as the foam stabilizer, a trimene base (ethane, chloro-, polymer with ammonia and formaldehyde) or Core Tex TNM-14S (polyalkylene glycol derivatives) may be used, but the foam stabilizer is not limited thereto.

In an exemplary embodiment of the present application, the foam stabilizer may be added in an amount of about 0.2 part by weight to about 1 part by weight based on the total weight 100 parts by weight of the cosmetic container in the composition, but the content of the foam stabilizer is not limited thereto. When the content of the foam stabilizer is less than about 0.2 part by weight, fine foams generated at the time of stirring the liquid composition may not be removed, and when the content is more than about 1 part by weight, the cosmetic container may be discolored, but the content is not limited thereto.

In an exemplary embodiment of the present application, the coagulant may include all types of coagulants typically used, but is not limited thereto. For example, as the coagulant, sodium silicofluoride or bentonite may be used, but the coagulant is not limited thereto.

In an exemplary embodiment of the present application, for the content of the coagulant, an amount of coagulant introduced may be adjusted depending on the coagulation rate. The coagulant may be added in an amount of about 0.5 part by weight to about 3 parts by weight based on the total weight 100 parts by weight of the cosmetic container in the composition, but the content of the coagulant is not limited thereto. There may occur problems in that when the content of the coagulant is less than about 0.5 part by weight, the mixtures of the liquid composition do not effectively coagulate, and when the content is more than about 3 parts by weight, the cosmetic container is caused to be aged, but the content is not limited thereto.

In an exemplary embodiment of the present application, the dispersant may include all types of dispersants typically used, but is not limited thereto. For example, as the dispersant, sodium butylnaphthalene sulfonate may be used, but the dispersant is not limited thereto.

In an exemplary embodiment of the present application, the dispersant may be added in an amount of about 0.2 part by weight to about 1 parts by weight based on the total weight 100 parts by weight of the cosmetic container in the composition, but the content of the dispersant is not limited thereto. When the content of the dispersant is less than about 0.2 part by weight, the composition mixtures of the cosmetic container are not uniformly dispersed, and when the content is more than about 1 part by weight, molecules of the dispersant are associated with one another to form spherical or rod-like micelles, thereby causing instability of particles of the nitrile butadiene rubber, but the content is not limited thereto.

In an exemplary embodiment of the present application, the cosmetic container may additionally include an antimicrobial agent, but is not limited thereto.

In an exemplary embodiment of the present application, as the antimicrobial agent, a gold-illite nano colloidal solution, a silver-tourmaline nano colloidal solution, and a metal nano colloidal solution coated with silicon oxide may be used, but the antimicrobial agent is not limited thereto. In the metal nano colloidal solution coated with silicon oxide, the metal may include those selected from the group consisting of gold (Au), silver (Ag), platinum (Pt), palladium (Pd), ruthenium (Ru), iron (Fe), copper (Cu), cobalt (Co), and nickel (Ni), but is not limited thereto. The antimicrobial agent may prevent inhabitation of bacteria which may be generated in the cosmetic container and prevent the skin inflammation from being induced through the suppression of bacteria, but the role of the antimicrobial agent is not limited thereto.

In an exemplary embodiment of the present application, the antimicrobial agent may be added in an amount of about 0.1 part by weight to about 0.5 part by weight based on the total weight of 100 parts by weight of the cosmetic container in the composition, but the content of the antimicrobial agent is not limited thereto. There may be problems in that when the content of the antimicrobial agent is less than about 0.1 part by weight, the antimicrobial action in the cosmetic container is weak, and when the content is more than about 0.5 part by weight, the pH and viscosity of the cosmetic container are decreased due to the content of the dispersant included in the antimicrobial agent particles themselves, foam are destroyed during foaming, and as a result, pores are not uniform, but the content is not limited thereto.

In an exemplary embodiment of the present application, the cosmetic composition may include an oily component, a thickener, a pigment, and an aqueous component, but the component is not limited thereto.

In an exemplary embodiment of the present application, the oily component may include those selected from the group consisting of a hydrocarbon, a vegetable oil, a synthetic ester, a silicone oil, and combinations thereof, but is not limited thereto.

In an exemplary embodiment of the present application, the pigment may include those selected from the group consisting of titanium dioxide, polymethylmethacrylate, silica, nylon, polyurethane, iron oxide, ultramarine, mica, synthetic mica, talc, pearl, sericite, boron nitride, and combinations thereof, but is not limited thereto.

In an exemplary embodiment of the present application, the aqueous component may include those selected from the group consisting of purified water, glycerin, propylene glycol, butylene glycol, pentylene glycol, phenoxyethanol, and combinations thereof, but is not limited thereto.

In an exemplary embodiment of the present application, the cosmetic composition with which the cosmetic container is filled includes a make-up base, a blemish balm (BB) cream, a complete correction (CC) cream, a foundation, a primer, a blusher, a lipstick, a lip gloss, a face powder, a lip liner, an eyebrow pencil, an eyeshadow, a compact powder, a twin cake, a pact, a powder pact, a concealer, or a sunscreen, but is not limited thereto.

In an exemplary embodiment of the present application, the cosmetic product may additionally include an applicator which applies the cosmetic composition, but is not limited thereto. The applicator may include those used as a use for applying the cosmetic composition without limitation, and may include, for example, a brush, a puff, a sponge, a roller, a stick, or the like, but is not limited thereto.

In an exemplary embodiment of the present application, the cosmetic product may additionally include a cosmetic vessel which accommodates the cosmetic composition, and a cosmetic container in which the cosmetic composition is filled, but is not limited thereto.

In an exemplary embodiment of the present application, the cosmetic product may additionally include an inner vessel which accommodates the cosmetic container in which the cosmetic composition is filled, and an outer vessel which accommodates the inner vessel, but is not limited thereto.

In an exemplary embodiment of the present application, the cosmetic product may be a pact type including: an applicator for application; a cosmetic container in which a cosmetic composition is filled; an inner vessel having the cosmetic container therein; and an external vessel which accommodates the inner container, in which the impregnation material includes the nitrile butadiene rubber according to an exemplary embodiment of the present application.

Hereinafter, the present application will be described in more detail by using the Examples, but the following Examples are exemplified to aid in understanding of the present application, and the content of the present application is not limited to the following Examples.

### Mode for Invention

### [Examples]

### 1. Experiments of Filling Efficiency and Stability According to Pore Size of NBR Sponge and Degree Effect of Release of Cosmetic Composition as Compared to Impregnation of Cosmetic Composition

### (1) Preparation of Samples in Examples and Comparative Examples

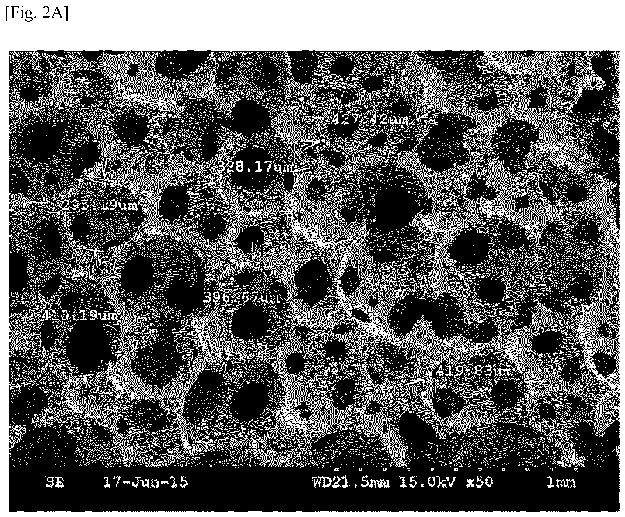

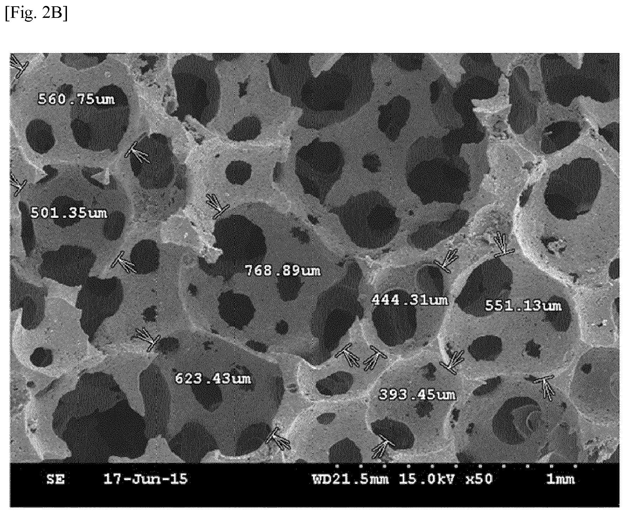

As described in the following [Table 1], NBR cosmetic containers having different sizes of pores were impregnated with about 14.5 g of each of the cosmetic compositions composed of the compositions described in the following [Table 1], and then stored in a thermostat at 25°C for a day or more. Here, an SEM image of the NBR sponge prepared according to Example 1 is illustrated in FIGS. 2A and 2B.

**[Table 1]**

| Classification | Name of raw material | Example 1 (parts by weight) | Comparative Example 1 (parts by weight) | Comparative Example 2 (parts by weight) |
|---|---|---|---|---|
| Oily component | Ethylhexyl methoxycinnamate | 6.7 | 6.7 | 6.7 |
| | Ethylhexyl salicylate | 4 | 4 | 4 |
| | Butylene glycol dicaprylate/Dicaprate | 5 | 5 | 5 |
| | Methyl trimethicone | 14 | 14 | 14 |
| | PEG-10 dimethicone | 0.5 | 0.5 | 0.5 |
| | Lauryl PEG-9 polydimethylsiloxyethyl dimethicone | 2 | 2 | 2 |
| | Tocopherol | | | |
| | Dimethicone | 3 | 3 | 3 |
| | Dimethicone crosspolymer | | | |
| | Acrylate/Stearyl acrylate/Dimethicone methacrylate copolymer | 0.5 | 0.5 | 0.5 |
| Thickener | Disteardimonium Hectorite | 0.2 | 0.2 | 0.2 |
| Pigment | Titanium dioxide | 4 | 4 | 4 |
| | Titanium dioxide | 7 | 7 | 7 |
| | Alumina | | | |
| | Stearic acid | | | |
| | Iron oxide yellow | 0.8 | 0.8 | 0.8 |
| | Iron oxide red | 0.2 | 0.2 | 0.2 |
| | Iron oxide black | 0.1 | 0.1 | 0.1 |
| | Mica | 4 | 4 | 4 |
| Aqueous component | Purified water | 40.5 | 40.5 | 40.5 |
| | Glycerin | 2 | 2 | 2 |
| | Butylene glycol | 3 | 3 | 3 |
| | Pentylene glycol | 1 | 1 | 1 |
| | Phenoxyethanol | 0.5 | 0.5 | 0.5 |
| | Salt | 1 | 1 | 1 |
| Applied sponge | | NBR | NBR | NBR |
| | | Pore size 0.5 mm | Pore size 0.15 mm | Pore size 1.2 mm |

### (2) Experimental Methods and Results Related To Filling Efficiency and

### Stability

The target filling amount was set to about 14.5 g by using a separately prepared filling facility. In the case of Comparative Example 1, the size of pores is so coarse and dense that the filling amount was about 10 g, and the filling efficiency was 68.9%, which was not good. In the case of Comparative Example 2, the target filling amount of about 14.5 g was completely filled, but the size of pores was so large that the cosmetic composition was discharged to the outside without being supported in the sponge, and accordingly, a result that it was difficult for the cosmetic composition to be stably supported occurred. In contrast, in the case of Example 1, the target filling amount of about 14.5 g was completely filled, the cosmetic composition was stably supported in the sponge while the filling efficiency was measured to be 100%, and the stability of the cosmetic composition may be secured.

### (3) Experiment on Effect of Discharge As Compared to Impregnation of Cosmetic Composition

### 1) Experimental Method

For each of the three samples prepared by filling the sponges in Example 1 and Comparative Example 1 with the cosmetic composition, the exhausted amount at each step, the total exhausted amount, and the exhausted amount as compared to the filling amount were measured respectively as follows by using a puff as an applicator.
(Step 1) An amount taken until almost no cosmetic composition came out by applying the cosmetic composition on white paper using a rubycell puff.
(Step 2) An amount taken until almost no cosmetic composition came out by flipping the rear surface of the sponge for impregnation in front, and then applying the cosmetic composition on white paper using a rubycell puff.
(Step 3) An amount taken until almost no cosmetic composition came out by taking out the sponge for impregnation and directly applying the cosmetic composition on white paper.

### 2) Experimental Results

The following [Table 2] is an experimental result according to the case of using the sponge in Example 1, and the following [Table 3] is an experimental result according to the case of using the sponge in Comparative Example 1.

**[Table 2]**

| Example 1 | Filling amount (g) | Exhausted amount (g) in Step 1 | Exhausted amount (g) in Step 2 | Exhausted amount (g) in Step 3 | Total exhausted amount (g) | Exhausted amount/filling amount (%) |
|---|---|---|---|---|---|---|
| Sample 1 | 14.5 | 8.95 | 1.79 | 1.31 | 12.05 | 83.1 |
| Sample 2 | 14.5 | 8.69 | 1.95 | 1.95 | 12.59 | 86.83 |
| Sample 3 | 14.5 | 9.26 | 2.06 | 0.65 | 11.97 | 82.55 |
| Average | | | | | 12.2 | 84.16 |

**[Table 3]**

| Comparative Example 1 | Filling amount (g) | Exhausted amount (g) in Step 1 | Exhausted amount (g) in Step 2 | Exhausted amount (g) in Step 3 | Total exhausted amount (g) | Exhausted amount/filling amount (%) |
|---|---|---|---|---|---|---|
| Sample 1 | 14.5 | 5.2 | 2.04 | 2.85 | 10.09 | 69.59 |
| Sample 2 | 14.5 | 3.39 | 2.1 | 4.25 | 9.74 | 67.17 |
| Sample 3 | 14.5 | 5.8 | 2.55 | 2.8 | 11.15 | 76.9 |
| Average | | | | | 10.33 | 71.22 |

As shown in Tables 2 and 3, the use efficiency in Comparative Example 1 was about 71%, which was exhibited as a considerably low numerical value, and the use efficiency in Example 1 was about 84% to about 86%, which was exhibited as a considerably high numerical value.

### 2. Sensory Comparison Evaluation of NBR Sponge and Polyurethane Sponge

### (1) Evaluation Method

The preference was evaluated for the pick-up amounts, close contact feeling, cover strength, texture of sponge, and spreadability in the NBR sponge according to Example 1 and the polyurethane sponge impregnated with the cosmetic composition which is the same as the cosmetic composition used in Example 1.

The preference for each sponge was performed with a questionnaire survey (30 subjects) consisting of a sensory evaluation (5 point scale method), and 5 points, 4 points, 3 points, 2 points, and 1 point were assigned to very good, good, fair, bad, and very bad, respectively.

### (2) Evaluation Result

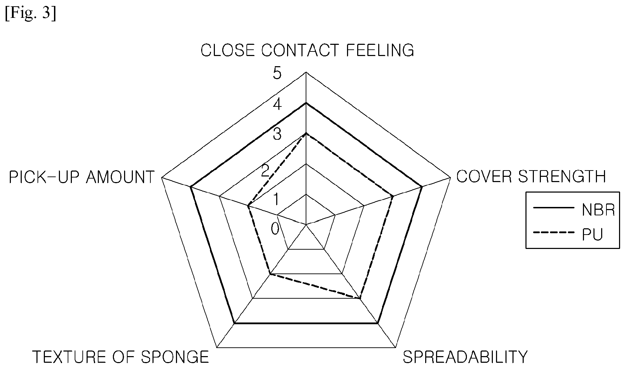

FIG. 3 is a graph illustrating a result of a sensory evaluation performed by using the NBR sponge and the polyurethane (PU) sponge, respectively. The NBR sponge was highly evaluated in most items during the sensory evaluation. In particular, the texture of the sponge and the pick-up amount of the content were highly evaluated. It was confirmed that in the case of the NBR sponge, a uniform content was discharged more continuously than the polyurethane sponge, and helped the effect of the make-up as the content was picked up in the puff.

### 3. Experiment Comparing Degree Effect of Release of Cosmetic Composition with Impregnation of NBR Sponge and Polyurethane Sponge with Cosmetic Composition

### (1) Experimental Example 1

### 1) Experimental Method

After a cosmetic composition having a viscosity of about 2,000 cps to about 3,000 cps was prepared by adjusting the content of the thickener in the cosmetic composition described in [Table 1], a polyurethane sponge having a pore size of about 0.5 mm and an NBR sponge having a pore size of about 0.5 mm (sponge in Example 1) were each filled with the cosmetic composition. After a vessel containing the filled sponges was stored upright for about 12 hours, the impregnation state of the impregnated content was observed.

### 2) Experimental Results

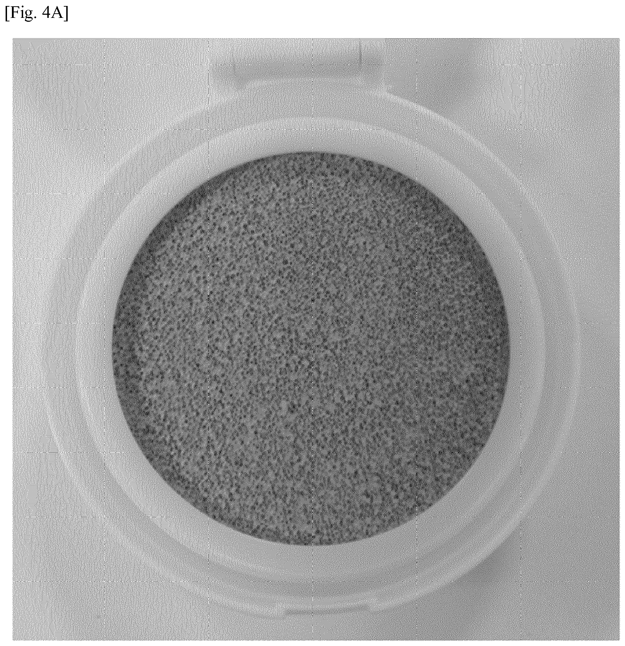

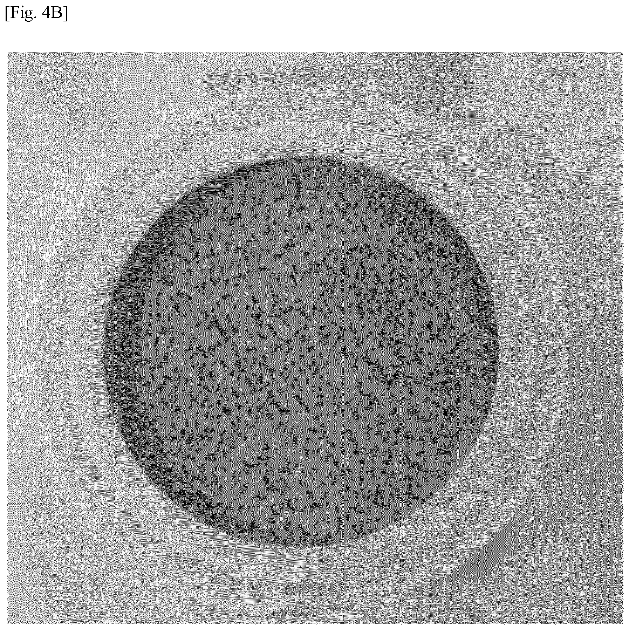

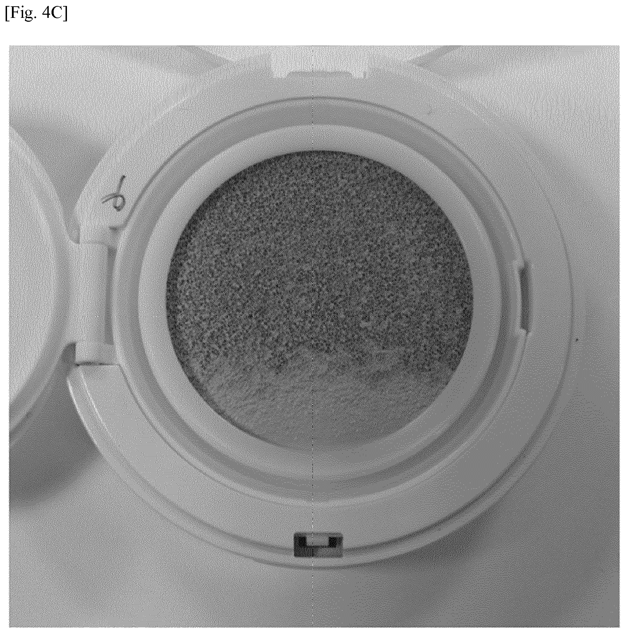

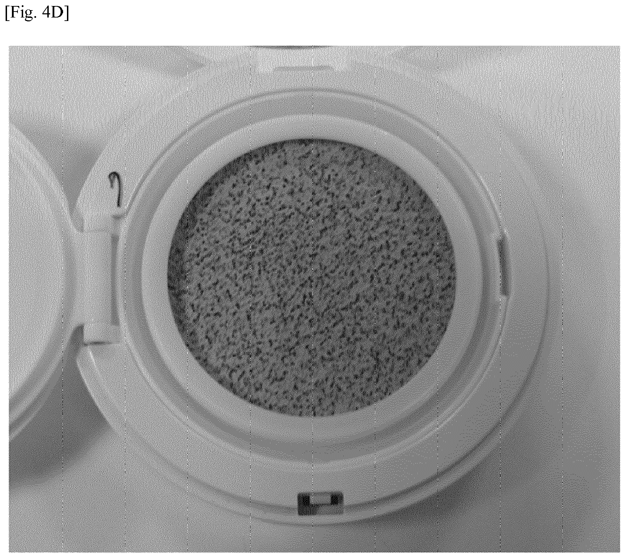

As can be confirmed in FIGS. 4A to 4D, it could be confirmed that as a result of confirming the impregnation state of the cosmetic composition after about 12 hours, the cosmetic composition leaked out of the polyurethane sponge without being stably supported in the sponge. In contrast, it was confirmed that the NBR sponge was impregnated with the same cosmetic composition, and even after 12 hours, the cosmetic composition was supported in the NBR sponge in the same state as immediately after the NBR sponge was impregnated with the cosmetic composition. Accordingly, it could be confirmed that in the maintenance of the stability of the low-viscosity cosmetic composition, the NBR sponge according to an Example of the present application was excellent.

### (2) Experimental Example 2

### 1) Experimental Method

After a cosmetic composition having a viscosity of about 6,500 cps was prepared by adjusting the content of the thickener in the cosmetic composition described in [Table 1], a polyurethane sponge having a pore size of about 0.5 mm and an NBR sponge having a pore size of about 0.5 mm (sponge in Example 1) were each filled with the cosmetic composition. After a vessel containing the filled sponges was stored upright at 45°C for 1 month, the impregnation state of the impregnated content was observed.

### 2) Experimental Results

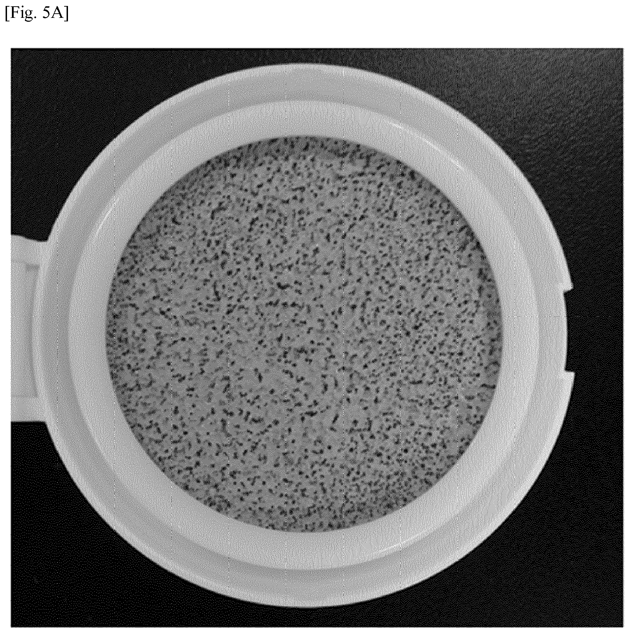

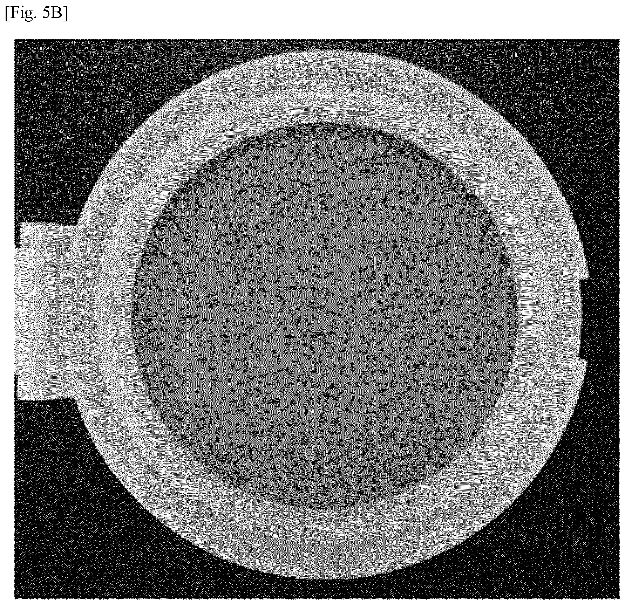

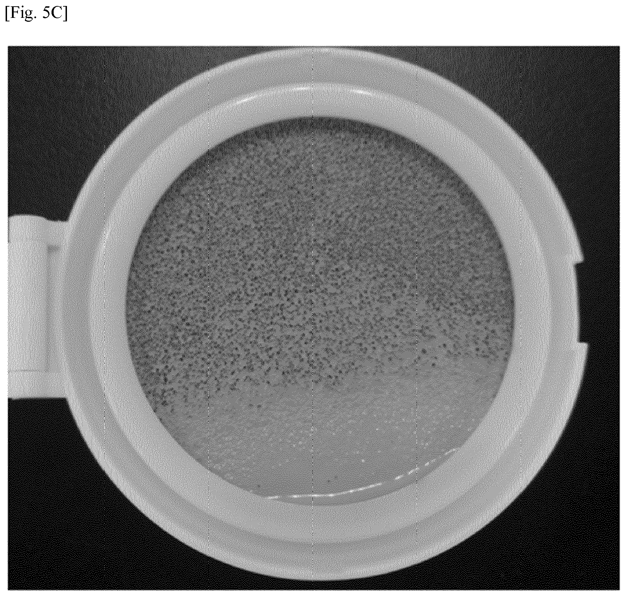

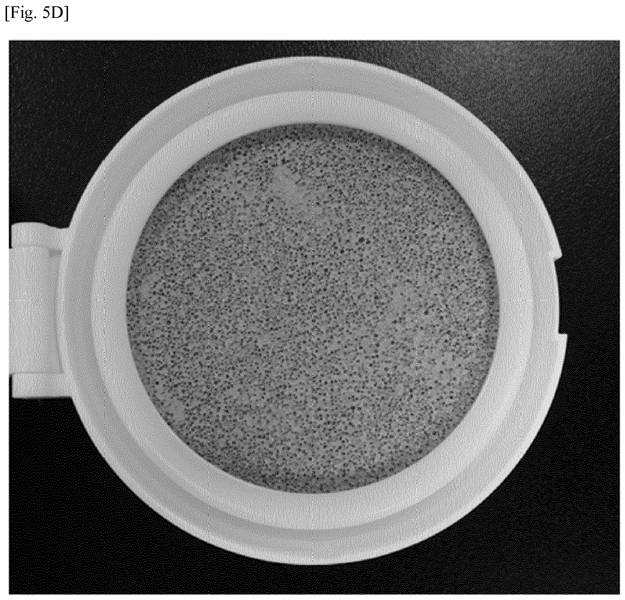

As can be confirmed in FIGS. 5A to 5D, it could be confirmed that as a result of confirming the impregnation state of the cosmetic composition after the cosmetic composition was stored at about 45°C for 1 month, the cosmetic composition leaked out of the polyurethane sponge without being stably supported in the sponge. In contrast, it was confirmed that the NBR sponge was impregnated with the same cosmetic composition, and even after the NBR sponge was stored at about 45°C for 1 month, the cosmetic composition was supported in the NBR sponge in the same state as immediately after the NBR sponge was impregnated with the cosmetic composition. Accordingly, it could be confirmed that in the maintenance of the stability of the low-viscosity cosmetic composition, the NBR sponge according to an Example of the present application was excellent.

The above-described illustration of the present application is provided for describing the present invention, and the person skilled in the art to which the present application pertains will understand that the present application can be easily modified into other specific forms without changing the technical spirit or essential features of the present application. Therefore, it should be understood that the above-described Examples are exemplary in all aspects and are not restrictive. For example, each constituent element which is described as a singular form may be implemented in a distributed form, and similarly, constituent elements which are described as being distributed may be implemented in a combined form.

The scope of the present application is represented by the claims to be described below rather than the detailed description, and it should be interpreted that the meaning and scope of the claims and all the changes or modified forms derived from the equivalent concepts thereto fall within the scope of the present application.

## Claims

1. A cosmetic product comprising:
a cosmetic composition; and
a cosmetic container in which the cosmetic composition is filled,
wherein the cosmetic container comprises nitrile butadiene rubber and has pores of which the average size is 0.2 mm to 1 mm, and
the cosmetic composition has a viscosity of 2,000 cps to 10,000 cps.

2. The cosmetic product of claim 1, wherein the cosmetic composition has a viscosity of 3,000 cps to 8,000 cps.

3. The cosmetic product of claim 1, wherein the cosmetic composition has a viscosity of 5,000 cps to 8,000 cps.

4. The cosmetic product of claim 1, wherein the nitrile butadiene rubber comprises a hydrophilic functional group.

5. The cosmetic product of claim 4, wherein the hydrophilic functional group is selected from the group consisting of -COOH, -SO₃, -PO₄H, -(CH₂)ₙSH (in the formula, n is an integer of 1 to 30), -((CH₂)₂O)ₙX (in the formula, X = hydrogen or an alkyl group, and n is an integer of 2 to 5), -OH, -SH, -O(CH₂S)ₙSH (in the formula, n is an integer of 1 to 30), -NH₂, and combinations thereof.

6. The cosmetic product of claim 1, wherein the cosmetic container comprises 10 ppi to 150 ppi of pores.

7. The cosmetic product of claim 1, wherein a hardness of the cosmetic container is 10 to 95 based on an ASKER hardness tester type F.

8. The cosmetic product of claim 1, wherein in the cosmetic container, the pores have an open cell structure.

9. The cosmetic product of claim 1, wherein the cosmetic container in which the cosmetic composition is filled has a filling efficiency of 75% or more.

10. The cosmetic product of claim 1, wherein a pick-up amount of cosmetic composition filling the cosmetic container is 0.3 g to 0.7 g per time.

11. The cosmetic product of claim 1, wherein the cosmetic container further comprises an antimicrobial agent.

12. The cosmetic product of claim 1, wherein the cosmetic composition comprises an oily component, a thickener, a pigment, and an aqueous component.

13. The cosmetic product of claim 12, wherein the oily component comprises oily components selected from the group consisting of a hydrocarbon, a vegetable oil, a synthetic ester, a silicone oil, and combinations thereof.

14. The cosmetic product of claim 12, wherein the pigment comprises pigments selected from the group consisting of titanium dioxide, polymethylmethacrylate, silica, nylon, polyurethane, iron oxide, ultramarine, mica, synthetic mica, talc, pearl, sericite, boron nitride, and combinations thereof.

15. The cosmetic product of claim 12, wherein the aqueous component comprises aqueous components selected from the group consisting of purified water, glycerin, propylene glycol, butylene glycol, pentylene glycol, phenoxyethanol, and combinations thereof.

16. The cosmetic product of claim 1, wherein the cosmetic composition comprises a foundation, a blemish balm (BB) cream, a complete correction (CC) cream, a make-up base, a primer, a blusher, a lipstick, a lip gloss, a face powder, a lip liner, an eyebrow pencil, an eyeshadow, a compact powder, a twin cake, a pact, a powder pact, a concealer, or a sunscreen.

17. The cosmetic product of claim 1, further comprising an applicator which applies the cosmetic composition.

18. The cosmetic product of claim 1, further comprising a cosmetic vessel which accommodates the cosmetic composition, and the cosmetic container in which the cosmetic composition is filled.
